# EUROPEAN PATENT APPLICATION

(11) **EP 4 129 176 A1**
(43) Date of publication of application: **08.02.2023**
(21) Application number: 21780568.8
(22) Date of filing: 25.03.2021
(51) Int. Cl.: A61B 5/25

(54) **BIOSENSOR**

(30) Priority: 30.03.2020 JP 2020059649
(71) Applicant: Nitto Denko Corporation, Ibaraki-shi, Osaka 567-8680 (JP)
(72) Inventor: YOSHIOKA, Ryoma, Ibaraki-shi, Osaka 567-8680 (JP); KONDO, Sota, Ibaraki-shi, Osaka 567-8680 (JP); ODANE, Chiharu, Ibaraki-shi, Osaka 567-8680 (JP)
(74) Representative: Hoffmann Eitle
(86) International application number: PCT/JP2021/012649
(87) International publication number: WO 2021/200575

(57) **Abstract**

A biological sensor includes a sensor body for acquiring biological information; an electrode connected to the sensor body; a first layer member configured to store the sensor body, a first adhesive layer being disposed on one surface of the first layer member facing the electrode; and a second layer member being stuck onto the one surface of the first layer member, and having a shape for covering the sensor body and exposing the electrode, a second adhesive layer being disposed on a surface of the second layer member opposite to the first layer member. A sticking surface onto a living body is formed by the first layer member and the second layer member.

## Description

### [Technical Field]

The present invention relates to biological sensors.

### [Background Art]

Wearable biological sensors that are attached to living bodies to acquire biological information such as electrocardiogram signals have been known. For example, such a type of biological sensor has an upper sheet, a lower sheet larger than the upper sheet, and an electric circuit unit disposed between the upper and lower sheets. The upper sheet is stuck onto the lower sheet in a state of holding the electric circuit unit between the upper sheet and the lower sheet, and the biological sensor is attached to skin by an adhesive layer provided on a side of the lower sheet opposite to the electric circuit unit (see, for example, Patent document 1).

### [Prior art documents]

### [Patent Documents]

[Patent Document 1] Japanese Patent No. 6537618

### [Summary of Invention]

### [Problem to be solved by the invention]

However, when the biological sensor is attached to the skin by a single lower sheet and a single adhesive layer, it is difficult to change an adhesion force depending on a position on the lower sheet, and to cause water repellence and moisture permeability of the lower sheet to coexist. For example, it is impossible to cause water repellence of a part that faces the electric circuit unit and moisture permeability of a part that does not face the electric circuit unit.

The present invention has been made in view of the above-described problem, and aims at providing a biological sensor capable of adjusting the adhesion force depending on a position of the lower sheet and causing water repellence and moisture permeability of the lower sheet to coexist, thereby preventing the biological sensor from being peeled off from a living body, improving a wearing feeling, and suppressing an occurrence of a failure due to water penetration into the sensor body.

### [Means for solving the problem]

According to an aspect of the present invention, a biological sensor includes a sensor body for acquiring biological information; an electrode connected to the sensor body; a first layer member configured to store the sensor body, a first adhesive layer being disposed on one surface of the first layer member facing the electrode; and a second layer member being stuck onto the one surface of the first layer member, and having a shape for covering the sensor body and exposing the electrode, a second adhesive layer being disposed on a surface of the second layer member opposite to the first layer member, a sticking surface onto a living body being formed by the first layer member and the second layer member.

### [Effects of the Invention]

According to the disclosed technique, it is possible to provide a biological sensor capable of adjusting the adhesion force depending on a position of the lower sheet and causing water repellence and moisture permeability of the lower sheet to coexist, thereby preventing the biological sensor from being peeled off from a living body, improving a wearing feeling, and suppressing an occurrence of a failure due to water penetration into the sensor body.

### [Brief Description of Drawings]

[FIGURE 1] FIG. 1 is a diagram depicting an example of a general arrangement of a biological sensor according to an embodiment of the present application.
[FIGURE 2] FIG. 2 is a plan view depicting an example of components of the biological sensor shown in FIG. 1.
[FIGURE 3] FIG. 3 is an exploded cross-sectional view depicting a cross section of the biological sensor shown in FIG. 1 cut along the longitudinal direction.
[FIGURE 4] FIG. 4 is a schematic view depicting the cross section of the biological sensor shown in FIG. 1 cut along the longitudinal direction.
[FIGURE 5] FIG. 5 is a diagram illustrating a state in which the biological sensor shown in FIG. 1 is attached to a chest of a living body.

### [Mode for carrying out the invention]

In the following, an embodiment for carrying out the invention will be described with reference to the drawings. In each drawing, the same reference numerals are assigned to the same components, respectively, and overlapping descriptions may be omitted.

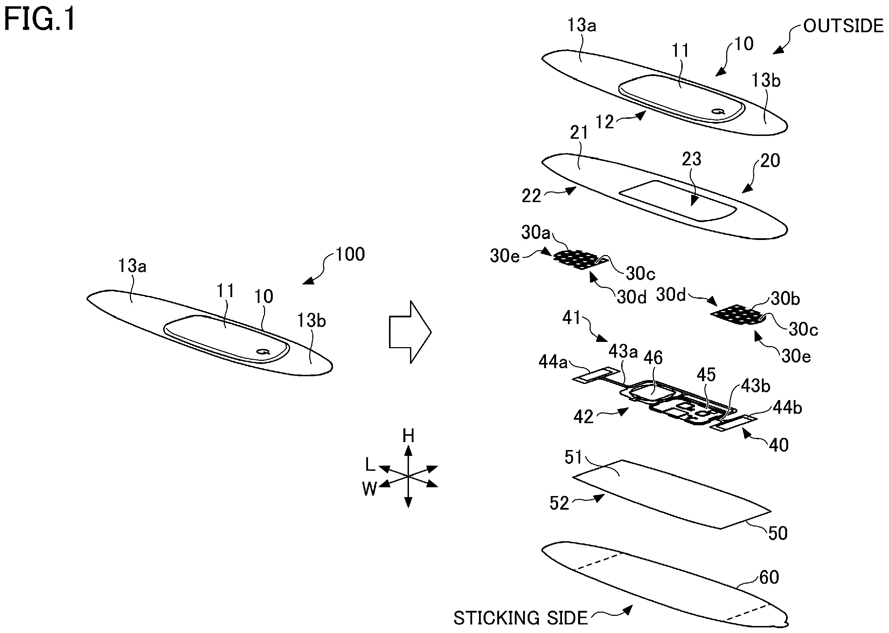

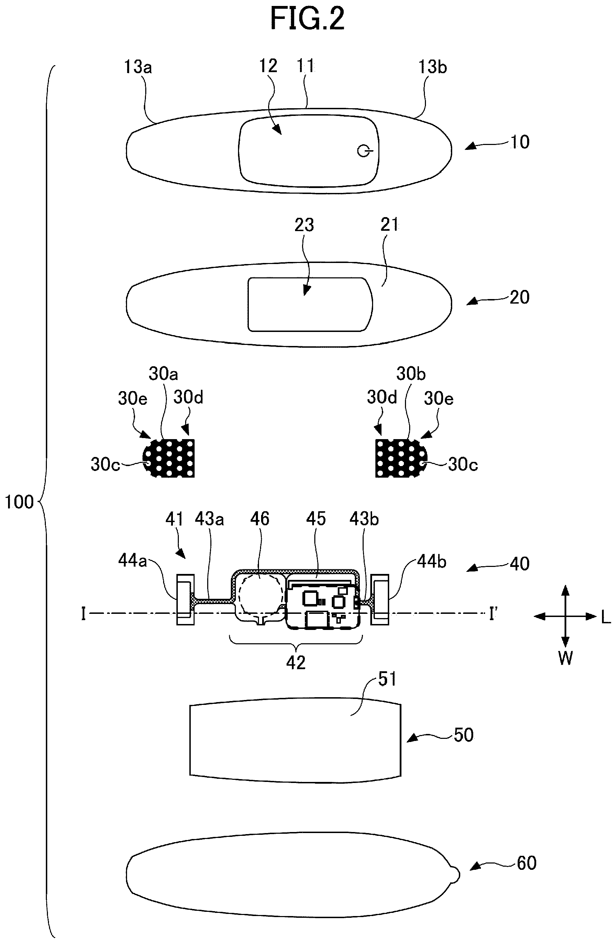

FIG. 1 is a diagram depicting an example of a general arrangement of a biological sensor according to an embodiment of the present application. A left part of FIG. 1 is an external view illustrating a biological sensor 100, and a right part of FIG. 1 illustrates the respective components of the biological sensor 100 exploded in the order of lamination. FIG. 2 is a plan view depicting an example of the components of the biological sensor shown in FIG. 1.

The biological sensor 100 shown in FIGS. 1 and 2 has an elongated shape and is formed by laminating a cover 10, an upper sheet 20, electrodes 30a and 30b, a sensor unit 40, a lower sheet 50 and a release sheet 60. As can be seen in FIGS. 1 and 2, each of the cover 10, the upper sheet 20, and the release sheet 60 has an elongated shape, and they have substantially the same shape. Moreover, an outer shape of the lower sheet 50 on both sides in the width direction W of the lower sheet 50 is almost the same as an outer shape of the upper sheet 20 on both sides in the width direction W.

In the following, when the electrodes 30a and 30b are described without distinction, they will be also referred to as an electrode 30. Moreover, in the following, a side (the release sheet 60 side) of the biological sensor 100 to be attached to a living body (a testee) will be referred to as a sticking side, and a side opposite to the sticking side (the cover 10 side) will be referred to as an outer side. The cover 10 and the upper sheet 20 are examples of a first layer member, and the lower sheet 50 is an example of a second layer member. The cover 10 is an example of a cover member, and the upper sheet 20 is an example of a foamed sheet.

The sensor unit 40 includes a flexible substrate 41 (resin substrate) on which various components for acquiring biological information are mounted. In the flexible substrate 41, a sensor body 42, constriction portions 43a and 43b, and terminal portions 44a and 44b connected to the sensor body 42 through the constriction portions 43a and 43b are integrally formed. In the following, the constriction portions 43a and 43b will be also referred to as a constriction portion 43 when the constriction portions 43a and 43b are described without distinction. Moreover, the terminal portions 44a and 44b will be also referred to as a terminal portion 44 when the terminal portions 44a and 44b are described without distinction.

The sensor body 42 includes a component mounting unit 45 and a battery mounting unit 46 to which a coin type battery or the like is mounted. The constriction portions 43a and 43b and terminal portions 44a, 44b are examples of connection portions. For example, a CR2025 battery is used as the battery.

The cover 10 is formed of a material having a flexibility such as, for example, a silicone resin (hardness: shore A 40). The cover 10 may be formed of a fluorinated resin (fluorinated rubber), a urethane resin (urethane rubber), or a styrene-butadiene rubber (SBR). The cover 10 has a projection portion 11 projecting outward in the height direction H in FIG. 1 in the central portion in the longitudinal direction L. The inside (sticking side) of the projection portion 11 is provided with a storage space 12 for storing the sensor body 42. The sticking side of the cover 10 has a flat shape.

Thicknesses of an upper surface and a sidewall of the projection portion 11 are thicker than thicknesses of flat portions 13a and 13b provided at both end sides in the longitudinal direction L of the cover 10. Thus, the flexibility of the projection portion 11 can be made smaller than the flexibility of the flat portions 13a and 13b, and the components mounted in the sensor body 42 can be protected from an external force applied to the biological sensor 100. Although the thicknesses of the upper surface and the side wall of the projection portion 11 are not particularly limited, the thicknesses are set, for example, within a range from 1.5 mm to 3 mm (millimeters), and the thicknesses of the flat portions 13a and 13b are set within a range from 0.5 mm to 1 mm.

On the other hand, the flat portions 13a and 13b which are thinner than the projection portion 11 are more flexible than the projection portion 11. Thus, when the biological sensor 100 is attached to the skin of a living body (testee), the flat portions 13a and 13b can be deformed following deformation of a body surface due to a body motion (stretching, bending, or twisting). Accordingly, when the body surface is deformed, a stress applied to the flat portions 13a and 13b can be reduced, so that the biological sensor 100 is not easily peeled off from the skin. In the following, the flat portions 13a and 13b will be also referred to as a flat portion 13, when the flat portions 13a and 13b are described without distinction.

Outer peripheries of the flat portions 13a and 13b are shaped so that thicknesses gradually decrease toward the ends. Thus, the flexibilities of the outer peripheries of the flat portions 13a and 13b can be made further higher, and the wearing feeling when the biological sensor 100 is attached to the living body P can be improved compared to a case where the thicknesses of the outer peripheries of the flat portions 13a and 13b are not reduced.

The upper sheet 20 is formed, for example, of a polyolefin-based foamed sheet of an open-cell structure, and has moisture permeability. On both surfaces of the upper sheet 20, adhesive layers 21 and 22 are provided, respectively. The adhesive layers 21 and 22 may be provided by applying double-sided adhesive tapes to the upper sheet 20, or by applying or spraying adhesive onto the upper sheet 20. The adhesive layer 22 is an example of a first adhesive layer, and the adhesive layer 21 is an example of a fourth adhesive layer.

For example, the adhesive used in the adhesive layer 22 provided on the sticking side of the upper sheet 20 has moisture permeability. Accordingly, as will be described below, water vapor generated by perspiration or the like generated from the living body, to which the biological sensor 100 is attached, may be discharged to the upper sheet 20 through the adhesive layer 22 and discharged from the upper sheet 20 to the outside of the biological sensor 100.

The thickness of the adhesive layer 22 may be changed depending on a position on the upper sheet 20. For example, the adhesive layer 22 may be formed by repeatedly arranging a strip (or a strip having no thickness) that is thinner than the other portions. In addition, the adhesive layer 22 may be formed so that the adhesive is dispersed, or the adhesive layer 22 may be formed so that non-adhesive portions are dispersed. The strip may have a line shape, a wave shape, or a circular shape. The thinner the adhesive is, the higher the moisture permeability of the adhesive layer 22 is. Therefore, by forming the adhesive layer 22 in which the adhesive is partially thin, the moisture permeability can be improved while maintaining the adhesion force.

Although the thickness of the upper sheet 20 is not particularly limited, the thickness of the upper sheet 20 is, for example, within a range from 0.5 mm to about 1.5 mm (preferably 1 mm). For example, FOLEC by INOAC Corporation is used for the upper sheet 20. For the adhesive layer 21, a ST503 (HC) 60 (thickness 55 um (micron)) by Nitto Denko Corporation is used, and a skin adhesive PANM (thickness 70 um) by Nitto Denko Corporation is used for the adhesive layer 22.

The upper sheet 20 has a through hole 23 at a position facing the sensor body 42. According to the through hole 23, the sensor body 42 can be stored in the storage space 12 of the cover 10 without being obstructed by the upper sheet 20.

An electrode 30 is, for example, a dry electrode that does not require application of a conductive gel when measuring a biological signal. The electrode 30 is formed by applying a conductive polymer to a resin sheet having a thickness of tens of micrometers (e.g. within a range from 20 um to 25 um). For example, polyethylene terephthalate with a thickness of 25 um is used for the resin sheet, and poly(3,4-ethylene dioxythiophene)-poly(styrene sulfonate) (PEDOT-PSS) is used for the conductive polymer. For the electrode 30, a single film of a conductive polymer without a resin sheet may be used. The electrode 30 is pasted to the upper sheet 20 via the adhesive layer 22 of the upper sheet 20.

The electrode 30 has a plurality of through holes 30c over the entire surface. One end (inside) of the electrode 30a in the longitudinal direction L is in contact with the terminal portion 44a, and one end (inside) of the electrode 30b in the longitudinal direction L is in contact with the terminal portion 44b. In the following, the end of the electrode 30a in contact with the terminal portion 44a and the end of the electrode 30b in contact with the terminal portion 44b will be referred to as facing portions 30d. In addition, a part of the electrode 30a that is not in contact with the terminal portion 44a and a part of the electrode 30b that is not in contact with the terminal portion 44b (the other ends (outside) in the longitudinal direction L) will be referred to as exposed portions 30e. The adhesive layer 22 can be exposed to the sticking side through the through holes 30c in the state where the electrode 30 is pasted to the adhesive layer 22.

The lower sheet 50 is formed, for example, of a resin sheet having a thickness being within a range from several tens of micrometers to about 100 um. Both surfaces of the lower sheet 50 are provided with adhesive layers 51 and 52, respectively. The adhesive layer 51 is an example of a third adhesive layer and the adhesive layer 52 is an example of a second adhesive layer. The resin sheet used for the lower sheet 50 is waterproof and impervious to moisture and vapor. The lower sheet 50 is formed in such a manner that the outer shapes of both sides in the width direction W are fit to the outer shapes of both sides in the width direction W of the upper sheet 20. For example, the lower sheet 50 is formed using ST254WB (thickness of 38 um) by Nitoms, Inc. For example, for the adhesive layer 51, SLY-258S-L (thickness of 50 µm) by Nitto Denko Corporation is used, and for the adhesive layer 52, Permeroll (thickness of 50 um) by Nitoms, Inc. is used.

A length of the lower sheet 50 in the longitudinal direction L is formed to be shorter than the upper sheet 20. Both ends of the lower sheet 50 in the longitudinal direction L are positioned so as to hold the terminal portions 44a and 44b between the lower sheet 50 and the upper sheet 20, and are formed in a position to expose the exposed portion 30e of the electrode 30. The sticking surface onto the living body P is formed by the lower sheet 50 and the upper sheet 20 extending from both ends of the lower sheet 50 in the longitudinal direction L. The adhesive layer 52 is provided on the sticking surface corresponding to the lower sheet 50, and the adhesive layer 22 is provided on the sticking surface corresponding to the upper sheet 20. Accordingly, water resistance/moisture permeability can be made different depending on the position on the sticking surface, and adhesion force can be made different.

Both ends of the lower sheet 50 in the longitudinal direction L of the adhesive layer 51 are disposed at positions facing the facing portion 30d of the electrode 30. Thus, the facing portion 30d of the electrode 30 and the terminal portion 44 can be held in a state of being pressed between the upper sheet 20 and the lower sheet 50. Then, the electrode 30 can be electrically connected to the terminal portion 44.

The release sheet 60 is stuck onto the adhesive layers 22 and 52, which are exposed to the sticking side until the biological sensor 100 is attached to the living body, in order to protect the adhesive layers 22 and 52 and the exposed portion 30e of the electrode 30.

The biological sensor 100 shown in FIGS. 1 and 2 is provided with the adhesive layers 21, 22, 51, and 52 only on the upper sheet 20 and the lower sheet 50. For example, a process requiring accuracy in pasting in order to prevent moisture from entering the sensor body 42 is the process of pasting the lower sheet 50 (adhesive layer 51) to the upper sheet 20 (adhesive layer 22) to which the electrodes 30 are pasted. For the biological sensor 100 according to the present embodiment, the number of pasting steps that require accuracy can be minimized, and misalignment during manufacturing (assembly) can be minimized. Accordingly, the manufacturing efficiency can be improved, reduction of the manufacturing yield, which is a good product ratio of the biological sensor 100, can be suppressed, and thereby the manufacturing cost can be reduced.

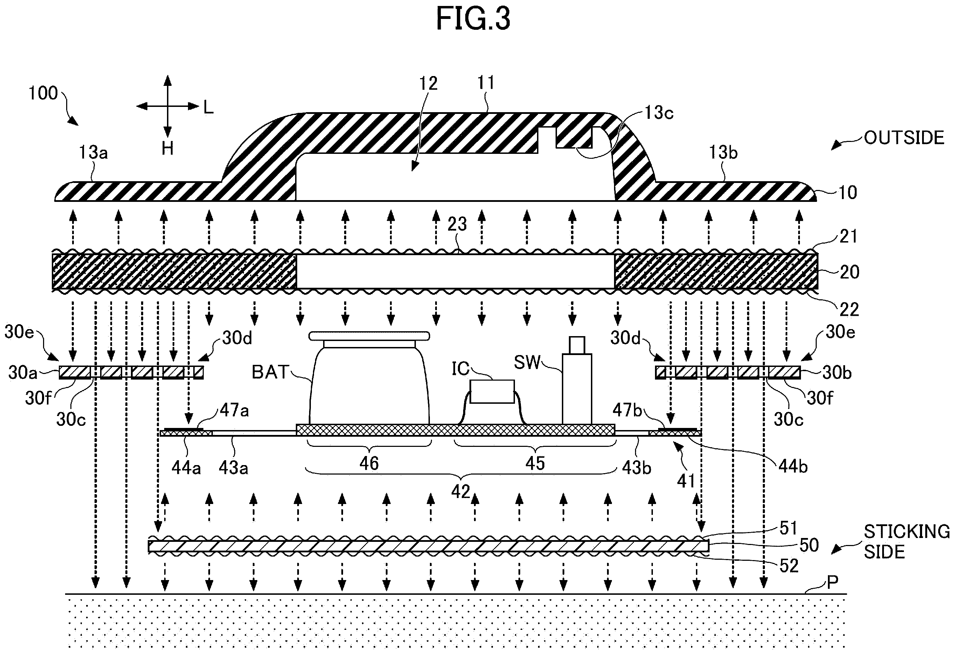

FIG. 3 is an exploded cross-sectional view depicting a cross section of the biological sensor 100 shown in FIG. 1 cut along the longitudinal direction L. FIG. 3 schematically shows the cross-section corresponding to the I-I' line shown in the sensor unit 40 of FIG. 2, and the cross-section is stretched emphasizing the height direction (thickness). The adhesive layers 21 and 22 provided on the upper sheet 20 and the adhesive layers 51 and 52 provided on the lower sheet 50 are indicated by wavy lines. Further, since FIG. 3 shows a state in which the biological sensor 100 is attached to the skin of the living body P (testee), the release sheet 60 of FIG. 1 has been removed.

In the present embodiment, the adhesion force of the adhesive layers 21 and 22 is higher than that of the adhesive layers 51 and 52. In FIG. 3, the adhesive layers 21 and 22 with relatively high adhesion forces are indicated by thick wavy lines, and the adhesive layers 51 and 52 with relatively low adhesion forces are indicated by thin wavy lines. Moreover, the thick dashed arrows shown in FIG. 3 in the vertical direction indicate that the adhesion force of the adhesive layers 21 and 22 is relatively high, and the thin dashed arrows indicate that the adhesion force of the adhesive layers 51 and 52 is relatively low. The adhesion force of the adhesive layer 51 may be higher than that of the adhesive layer 52, and may be almost the same as that of the adhesive layer 22.

The adhesive layer 52 of the lower sheet 50 is attached to the skin of the living body P to fix the biological sensor 100 to the living body P. A part of the biological sensor 100 outside the terminal portion 44 in the longitudinal direction L is attached to the skin by the high adhesion force of the adhesive layer 22, and a part of the biological sensor 100 inside the terminal portion 44 in the longitudinal direction L is attached to the skin by the low adhesion force of the adhesive layer 52.

Via the adhesive layer 21 of the upper sheet 20, the upper sheet 20 is stuck onto a flat surface on the sticking side of the cover 10. A portion of the adhesive layer 22 of the upper sheet 20 facing the electrode 30 (30a, 30b) is pasted to the electrode 30. A portion of the adhesive layer 22 facing the through hole 30c of the electrode 30 is exposed to the sticking side through the through hole 30c.

The adhesive layer 22 exposed from the through hole 30c provided in the exposed portion 30e of the electrode 30 is attached to the living body P and functions to bring the electrode 30 into close contact with the skin of the living body P. The adhesive layer 22 exposed from the through holes 30c provided in the facing portions 30d of the electrode 30 is pasted to pads 47 (47a and 47b) of the terminal portion 44 (44a and 44b) and functions to bring the electrode 30 into close contact with the pads 47.

For example, a surface of the electrode 30 on the sticking side is provided with an electrically conductive polymer 30f, and the surface of the pad 47 is plated with gold. The conductive polymer 30f may be provided at least on the surface of the electrode 30 on the sticking side, but may be provided on both surfaces.

Within the adhesive layer 22 facing neither the flexible substrate 41 nor the electrode 30, a portion facing the lower sheet 50 is pasted to the adhesive layer 51 of the lower sheet 50. According to the pasting of the adhesive layer 22 to the adhesive layer 51, the conductive polymer 30f of the electrode 30 and the pads 47a of the terminal portion 44 are brought into close contact with each other in a state of being pressed. On the other hand, within the adhesive layer 22 facing neither the flexible substrate 41 nor the electrode 30, a portion that does not face the lower sheet 50 is attached to the skin of the living body P.

For example, the sensor body 42 includes an integrated circuit IC, such as a CPU or ASIC, which processes a biological signal acquired from the living body P to generate biological signal data; a switch SW that activates the biological sensor 100; and a battery BAT that supplies power to the integrated circuit IC. The integrated circuit IC and the switch SW are mounted on a component mounting unit 45 and the battery BAT is mounted on a battery mounting unit 46. For example, the switch SW is a push-down switch. In the storage space 12, at a position facing the switch SW, a protrusion 13c is formed so as to reduce a distance from an end of the switch SW and apply a pressing force from the projection portion 11 side to the end of the switch SW without dispersing the pressing force.

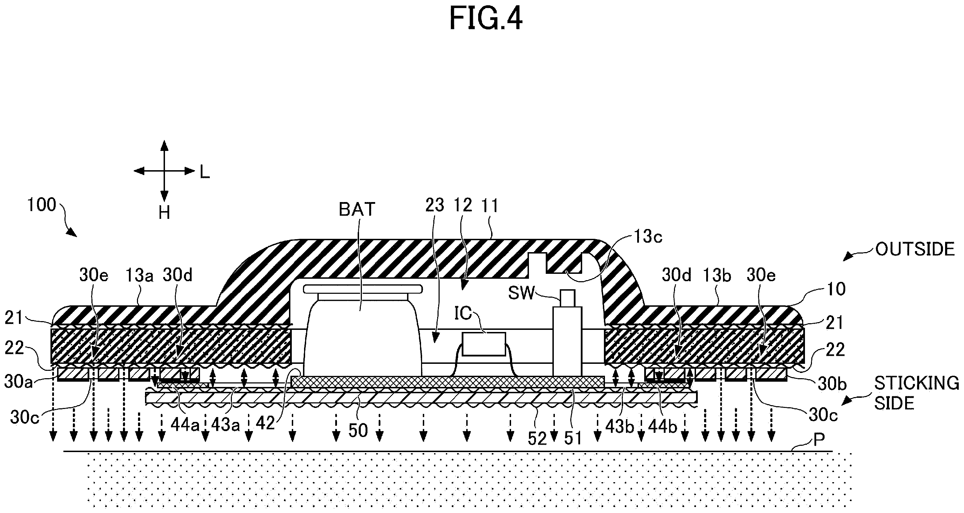

FIG. 4 is a schematic diagram illustrating a cross-section of the biological sensor 100 shown in FIG. 1 cut along the longitudinal direction L. The same elements as those in FIG. 3 are indicated by the same reference numerals. FIG. 4 schematically illustrates the cross-section corresponding to the I-I' line shown in the sensor unit 40 in FIG. 2, in the same manner as FIG. 3, and stretches the cross-section emphasizing the height direction (thickness).

In the present embodiment, since the adhesive layer 22 of the upper sheet 20 is moisture permeable, water vapor generated from the living body P, to which the biological sensor 100 is attached, can be discharged to the upper sheet 20 via the adhesive layer 22. Additionally, the upper sheet 20 has an open-cell structure so that water vapor entering through the adhesive layer 22 can be discharged to the outside of the biological sensor 100.

According to the above-described feature, it is possible to suppress accumulation of perspiration or water vapor at an interface between the skin of the living body P, to which the biological sensor 100 is attached, and the adhesive layer 22. As a result, it is possible to prevent the biological sensor 100 from being peeled off from the skin due to the moisture accumulated at the interface between the skin and the adhesive layer 22 that reduces the adhesion force of the adhesive layer 22.

On the other hand, the lower sheet 50 is formed of a waterproof resin sheet. Thus, it is possible to prevent perspiration or water vapor generated from the living body P from entering the flexible substrate 41 side through the lower sheet 50 in the state where the biological sensor 100 is attached to the skin of the living body P. Moreover, the electrodes 30 (30a, 30b) and terminal portions 44 (44a, 44b) are disposed between the upper sheet 20 and the lower sheet 50, and are brought into contact with each other in a pressing state by the adhesive layers 22 and 51. Thus, it is possible to prevent perspiration or water vapor from the interface between the lower sheet 50 and the terminal portion 44 from entering the sensor body 42 .

As shown in FIG. 4, the interface between the ends of the terminal portions 44 and the electrodes 30 may be covered by the adhesive layer 51 by protruding both ends of the lower sheet 50 in the longitudinal direction L from the ends of the terminal portions 44. According to the above-described feature, it is possible to prevent perspiration or water vapor from entering the sensor body 42 from the interface between the terminal portion 44 and the electrode 30.

Moreover, because the adhesive layer 22 of the upper sheet 20 is exposed to the terminal portion 44 side through the through hole 30c provided in the electrode 30, the pressure force to the electrode 30 can be maintained over the entire surface of the terminal portion 44. Thus, it is possible to block the path of perspiration or water vapor entering the sensor body 42 from the interface between the lower sheet 50 and the terminal portion 44 and the interface between the terminal portion 44 and the electrode 30.

According to the above-described structure, it is possible to prevent a failure or disconnection of a component, such as the integrated circuit IC mounted in the component mounting unit 45, the battery BAT mounted in the battery mounting unit 46, or a wiring, due to corrosion or the like. As a result, it is possible to prevent the biological sensor 100 from not operating normally, and it is possible to suppress the biological signal from being unable to be measured.

The electrode 30 and the terminal portion 44 are held between the upper sheet 20 and the lower sheet 50 via the adhesive layers 22 and 51. Therefore, the adhesion forces of the adhesive layers 22 and 51 can be used to bring the facing portions 30d of the electrodes 30 and the terminal portions 44 into contact with each other in a state of being pressed, and thereby a contact resistance between the electrodes 30 and the terminal portions 44 can be reduced.

The facing portions 30d of the electrode 30 can be brought into contact with the terminal portions 44 in the state of being pressed, by the adhesive layer 22 which is exposed via the through holes 30c located in the facing portions 30d of the electrode 30. Thus, the contact resistance between the electrode 30 and the terminal portion 44 can be further reduced compared with the case where the electrode without the through hole 30c is brought into contact with the terminal portion 44.

Moreover, according to the adhesive layer 22 located around the exposed portion 30e, the exposed portion 30e of the electrode 30 can be brought into contact with the skin of the living body P in a state of being pressed. Furthermore, the exposed portions 30e of the electrodes 30 can be pressed into contact with the skin of the living body P by the adhesive layer 22 which is exposed through the through hole 30c located in the exposed portions 30e of the electrodes 30. Therefore, it is possible to increase the pressure force on the skin not only at the periphery of the exposed portion 30e but also at the center of the exposed portion 30e.

As described above, it is possible to reduce the contact resistance between the electrode 30 and the terminal portion 44, by holding the electrode 30 and the terminal portion 44 between the upper sheet 20 and the lower sheet 50 via the adhesive layers 22 and 51. Moreover, by the adhesive layer 22 around the electrode 30 and the adhesive layer 22 exposed through the through-hole 30c, the electrode 30 can be brought into contact with the skin in the state of being pressed, and thereby the contact resistance between the electrode 30 and the skin can be reduced. As a result, the detection accuracy of the biological signal by the biological sensor 100 can be enhanced.

In addition, when the contact resistance between the electrode 30 and the terminal portion 44 and the contact resistance between the electrode 30 and the living body P can be suppressed to less than or equal to predetermined values respectively, the through hole 30c may not be provided in the electrode 30. Alternatively, the through hole 30c may be provided in only one of the facing portions 30d and the exposed portions 30e of the electrode 30 based on the evaluation for the contact resistance value.

The adhesive layer 51 of the lower sheet 50 is stuck onto the upper sheet 20 over the constriction portion 43 disposed in a flat manner along the lower sheet 50, and secures the constriction portion 43 in a state of being held between the upper sheet 20 and the lower sheet 50. Because the upper sheet 20 has the through hole 23 through which the sensor body 42 can be inserted, it is possible to hold the constriction portion 43 between the upper sheet 20 and the lower sheet 50 without bending the upper sheet 20 in the height direction H by arranging the sensor body 42 on the sticking side of the upper sheet 20.

Furthermore, the adhesive layer 51 of the lower sheet 50 is stuck onto a flat surface of the sensor body 42 on the sticking side, and secures the sensor body 42 in a state where the sensor body 42 is stored in the storage space 12 which is provided in the cover 10. Thus, even when the biological sensor 100 vibrates due to body movement of the living body P to which the biological sensor 100 is attached, the sensor body 42 and the constriction portion 43 can be integrally vibrated, and thereby concentration of stress into the constriction portion 43 can be suppressed. As a result, disconnection due to deformation of the wiring of the constriction portion 43 can be suppressed.

By strengthening the adhesion force of the adhesive layer 22 located at both end sides in the longitudinal direction L, it is possible to prevent the adhesive layer 22 from being peeled off from the skin, even when a stress is applied on the sticking surface of the biological sensor 100 to the skin due to the body movement of the living body P. Accordingly, it is possible to suppress an increase in the contact resistance between the electrode 30 and the skin, and it is possible to suppress a decrease in the accuracy of the measurement of the biological signal.

Because the adhesion force of the adhesive layer 52 is weak, the pain upon peeling the biological sensor 100 from the living body P can be reduced. As a result, while suppressing the pain upon peeling the biological sensor 100 from the living body P, it is possible to prevent the accuracy of the measurement when the biological signal is measured from being degraded, or it is possible to prevent the biological signal from becoming unable to be measured.

Because the upper sheet 20 is formed of a foamed sheet, the upper sheet 20 can absorb a part of the stress, applied to the sticking surface of the biological sensor 100, to the skin due to deformation of the skin by movement of the living body P. By reducing the stress, applied to the sticking surface, to the skin, it is possible to reduce a sense of tightness felt by the living body P when the skin is deformed, thereby the wearing feeling when the biological sensor 100 is attached to the living body P can be improved.

The improvement of the wearing feeling when the biological sensor 100 is attached to the living body P can be obtained by forming the upper sheet 20 with a foamed material having flexibility. However, the improvement also can be obtained by reducing the thickness of the peripheral edge of the cover 10 (in particular, the thickness of both sides in the longitudinal direction L). By forming the upper sheet 20 with the foamed material and reducing the thickness of the peripheral edge of the cover 10, it is possible to improve the wearing feeling of the biological sensor 100 when the biological sensor 100 is attached to the living body P.

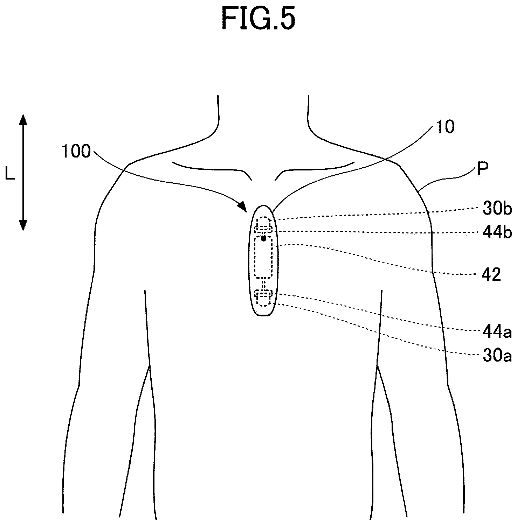

FIG. 5 is an explanatory diagram illustrating a state where the biological sensor 100 shown in FIG. 1 is attached to a chest of the living body P. For example, the longitudinal direction L of the biological sensor 100 is aligned with the sternum of the living body P, and the biological sensor 100 is attached to the living body P with the electrode 30b being on the upper side and the electrode 30a being on the lower side of the living body P. The biological sensor 100 acquires a biological signal, such as an electrocardiogram signal, from the living body P in the state where the electrodes 30a and 30b are pressed into contact with the body surface of the living body P by sticking the adhesive layers 22 and 52 shown in FIG. 4 onto the living body P. For example, the biological sensor 100 stores the acquired biological signal data in a non-volatile memory such as a flash memory mounted in the component mounting unit 45.

As described above, in the embodiments shown in FIGS. 1 to 5, the biological sensor 100 has an upper sheet 20 and a lower sheet 50 that differ in adhesion force and moisture permeability (waterproof). Thus, the adhesion force to the living body P and the permeability of moisture can be changed, for example, according to the portion where the electrode 30 is formed or the portion where the component is mounted, and the adhesion force and the permeability (waterproof) can be set depending on the position in the biological sensor 100. As a result, it is possible to provide the biological sensor 100 that can release water generated from the living body P and prevent water from entering the sensor body 42.

As a result, it is possible to prevent the biological sensor 100 from being peeled off from the living body P, and at the same time, it is possible to prevent failure of the biological sensor 100 due to water penetration into the sensor body 42 from occurring. In other words, it is possible to prevent the biological signal from becoming unable to be measured due to perspiration or water vapor generated from the living body to which the biological sensor is attached.

Because the number of pasting steps that require accuracy in order to suppress entering of moisture into the sensor body 42 can be minimized, the manufacturing efficiency in the assembly process of the biological sensor 100 can be improved. Thus, it is possible to suppress the reduction of the manufacturing yield, which is a good product ratio of the biological sensor 100, and thereby the manufacturing cost can be reduced.

Because the through hole 23 through which the sensor body 42 can be inserted is provided in the upper sheet 20, and the sensor body 42 is disposed on the sticking side of the upper sheet 20, the constriction portion 43 can be held between the upper sheet 20 and the lower sheet 50 without bending in the height direction H. Accordingly, it is possible to prevent mechanical stress from being applied to the constriction portion 43 in a bending state, and thereby breakage of wire of the constriction portion 43 can be suppressed.

By holding the electrode 30 and the terminal portion 44 by the adhesive layers 22 and 51 in a state of being pressed, it is possible to prevent perspiration or water vapor from entering the sensor body 42 from the interface between the electrode 30 and the terminal portion 44. As a result, an occurrence of a failure of the component mounted on the sensor body 42 can be suppressed, and it is possible to prevent the biological sensor 100 from becoming unable to operate normally.

Because the upper sheet 20 is a flexible foamed sheet, a part of the stress applied to the sticking surface of the biological sensor 100 to the skin due to the body movement (stretching, bending, or twisting) of the living body P can be absorbed by the upper sheet 20. Thus, the wearing feeling of the biological sensor 100 when the biological sensor 100 is attached to the living body P can be improved. Moreover, by the upper sheet 20, which is a foamed sheet of an open-cell structure, moisture generated from the living body P can be efficiently discharged, the moisture can be prevented from accumulating at the interface between the adhesive layer 22 and the skin, and the biological sensor 100 can be prevented from being peeled off from the skin. As a result, it is possible to prevent the biological sensor 100 attached to the living body P from becoming unable to measure biological signals.

By strengthening the adhesive force of the adhesive layer 22 outside the sensor body 42 in the longitudinal direction L, even in a case of stress, applied to the sticking surface of the biological sensor 100, to the skin due to the body movement of the living body P, it is possible to prevent the adhesive layer 22 from being peeled off from the skin. Accordingly, it is possible to suppress an increase in the contact resistance between the electrode 30 and the skin, and suppress a decrease in an accuracy of the measurement of the biological signal.

Because the adhesion force of the adhesive layer 52 is weak, the pain upon peeling the biological sensor 100 from the living body P can be reduced. As a result, while suppressing the pain upon peeling the biological sensor 100 from the living body P, it is possible to prevent the accuracy of the measurement when the biological signal is measured from being degraded, or it is possible to prevent the biological signal from becoming unable to be measured.

By making the thicknesses of the flat portions 13a and 13b thinner than the thicknesses of the projection portions 11, the flat portions 13a and 13b can be deformed following deformation of a body surface due to a body motion of the living body P to which the biological sensor 100 is attached. Accordingly, the stress applied to the flat portions 13a and 13b due to the body motion can be reduced, and the biological sensor 100 can be prevented from being peeled off from the skin.

By exposing the adhesive layer 22 through the through hole 30c of the electrode 30 to the sticking side, the exposed portion 30e of the electrode 30 can be pressed into contact with the skin of the living body P. Therefore, it is possible to increase the pressure force to the skin not only at the periphery of the exposed portion 30e but also at the center of the exposed portion 30e. As a result, contact resistance between the electrode 30 and the skin can be reduced, and thereby the accuracy of detection of the biological signal by the biological sensor 100 is further improved.

Moreover, by exposing the adhesive layer 22 through the through hole 30c of the electrode 30 to the sticking side, the facing portions 30d of the electrode 30 can be brought into contact with the terminal portion 44 in a state of being pressed by the adhesive layer 22 exposed through the through hole 30c. As a result, the contact resistance between the electrode 30 and the terminal portion 44 can be further reduced compared to the case where the electrode without the through hole 30c is brought into contact with the terminal portion 44, and thereby the detection accuracy of the biological signal by the biological sensor 100 can be further enhanced.

As described above, the present invention has been described according to the embodiments. However, the present invention is not limited to the above-described specifically disclosed embodiments. In these respects, various modifications may be made without departing from the scope of the present invention.

The present international application claims the priority based on Japanese Patent Application No. 2020-059649, filed March 30, 2020, and the entire content of Japanese Patent Application No. 2020-059649 is incorporated herein by reference.

### [Reference signs list]

10 Cover
11 Projection portion
12 Storage space
13(13a,13b) Flat portion
20 Upper sheet
21,22 Adhesive layer
23 Through hole
30(30a,30b) Electrode
30c Through hole
30d Facing portion
30e Exposed portion
30f Conductive polymer
40 Sensor unit
41 Flexible substrate
42 Sensor body
43(43a,43b) Constriction portion
44(44a,44b) Terminal portion
45 Component mounting unit
46 Battery mounting unit
47(47a,47b) Pad
50 Lower sheet
51,52 Adhesive layer
60 Release sheet
100 Biological sensor
BAT Battery
IC Integrated circuit
P Living body
SW Switch

## Claims

1. A biological sensor comprising:
a sensor body for acquiring biological information;
an electrode connected to the sensor body;
a first layer member configured to store the sensor body, a first adhesive layer being disposed on one surface of the first layer member facing the electrode; and
a second layer member being stuck onto the one surface of the first layer member, and having a shape for covering the sensor body and exposing the electrode, a second adhesive layer being disposed on a surface of the second layer member opposite to the first layer member,
wherein a sticking surface onto a living body is formed by the first layer member and the second layer member.

2. The biological sensor according to claim 1 further comprising:
a connection portion disposed between the first layer member and the second layer member, overlapping with a part of the electrode, and for connecting the electrode to the sensor body.

3. The biological sensor according to claim 2 further comprising:
a third adhesive layer disposed on the second layer member at least at a position facing a part in which the connection portion overlaps with the electrode.

4. The biological sensor according to claim 2 or 3,
wherein the connection portion is disposed between the electrode and the second layer member, and includes a terminal portion being in contact with the electrode, and
wherein the electrode includes a through hole, the first adhesive layer being exposed to the connection portion through the through hole in a state where the electrode is stuck onto the first adhesive layer.

5. The biological sensor according to any one of claims 1 to 4,
wherein the first layer member includes
a cover member including a storage space for storing the sensor body;
a foamed sheet including a through hole at a position corresponding to the storage space; and
a fourth adhesive layer for pasting the cover member and the foamed sheet to each other,
wherein the first adhesive layer is disposed on the second layer member side of the foamed sheet.

6. The biological sensor according to claim 5,
wherein the foamed sheet has an open-cell structure.

7. The biological sensor according to claim 5 or 6,
wherein the cover member and the first layer member have elongated shapes,
wherein the electrodes are disposed at both end sides in a longitudinal direction,
wherein the sensor body is disposed in a central portion in the longitudinal direction, and
wherein thicknesses of the cover member at the both end sides in the longitudinal direction are less than a thickness of the cover member in the central portion in the longitudinal direction.

8. The biological sensor according to any one of claims 1 to 7,
wherein the electrode includes a through hole, the first adhesive layer being exposed through the through hole in a state where the electrode is stuck onto the first adhesive layer.

9. The biological sensor according to any one of claims 1 to 8,
wherein a thickness of the first adhesive layer is changed depending on a position on the first layer member.

10. The biological sensor according to any one of claims 1 to 9,
wherein an adhesive force of the first adhesive layer is stronger than an adhesive force of the second adhesive layer.

11. The biological sensor according to any one of claims 1 to 10,
wherein the second layer member is waterproof.
